# EUROPEAN PATENT SPECIFICATION

(11) **EP 0 529 197 B1**
(45) Date of publication and mention of the grant of the patent: **07.01.1998**
(21) Application number: 92106244.4
(22) Date of filing: 10.04.1992
(51) Int. Cl.: C07D 303/16

(54) **A process for the preparation of a purified 3,4-epoxycyclohexyl-methyl (meth)acrylate .**
Verfahren zur Herstellung von gereinigtem 3,4-Epoxycyclohexyl-Methyl (Meth)Acrylat .
Procédé pour la préparation de 3,4-époxycyclohéxyl méthyl (méth)acrylate purifié .

(30) Priority: 28.08.1991 JP 216798/91; 05.12.1991 JP 321724/91
(43) Date of publication of application: 03.03.1993
(60) Divisional application: 96111772.8
(73) Proprietor: DAICEL CHEMICAL INDUSTRIES, LTD., Sakai-shi Osaka-fu 590 (JP)
(72) Inventor: Kuwana, Akihiro, Otake-shi, Hiroshima-ken (JP); Honda, Kimihide, Himeji-shi, Hyogo-ken (JP); Koga, Kunio, Himeji-shi, Hyogo-ken (JP)
(74) Representative: Hansen, Bernd, Dr. Dipl.-Chem.

(56) References cited:
- EP-A- 0 212 210
- DATABASE WPIL Week 8532, Derwent Publications Ltd., London, GB; AN 85-194703
- CHEMICAL ABSTRACTS, vol. 114, 1991, Columbus, Ohio, US; abstract no. 165087
- CHEMICAL ABSTRACTS, vol. 96, 1982, Columbus, Ohio, US; abstract no. 162451

## Description

### FIELD OF THE INVENTION

The present invention relates to a purified 3,4-epoxycyclohexyl methyl (meth)acrylate, a process for the preparation thereof, and a 3,4-epoxycyclohexyl methyl (meth)acrylate composition.

In particular, the present invention relates to the improvements in a process for the preparation of a purified 3,4-epoxycyclohexyl methyl (meth)acrylate.

### BACKGROUND OF THE INVENTION

Heretofore, various acrylate monomers such as methylacrylate, ethylacrylate, 2-ethylhexyl acrylate, etc., which are monofunctional monomers, and trimethylolpropane triacrylate, pentaerythritol triacrylate, etc., which are multifunctional monomers have been widely known.

However, the monofunctional monomers have a disadvantage in that an odor of the residual monomer remaining after curing causes a remarkable problem when the monomers are used as a component of printing inks or coatings.

Furthermore, the multifunctional monomers also have a disadvantage that it is necessary to use large amounts of monomers to resins when using the monomer as a diluent of printing inks or coatings, resulting in loss of excellent properties of the resins.

On the other hand, cyclohexyl methyl (meth)acrylate itself is readily polymerized or copolymerized with other compounds having unsaturated groups by heat, ultraviolet rays and or ionized radiation in the presence of an initiator for free radical polymerization.

In particular, an epoxidized cyclohexyl methyl (meth)acrylate which has an alicyclic epoxy group capable of being cured by a cation, that is, 3,4-epoxycyclohexyl methyl (meth)acrylate is useful for polymerizing or copolymerizing.

3,4-epoxycyclohexyl methyl (meth) acrylate is low in viscosity and mild in odor, and has a wide range of solubility to resins, and further it is useful for inks, coatings, adhesives, covering agents and as a raw material for molding resins or a modifier thereof.

It is noted that 3,4-epoxycyclohexyl methyl (meth)acrylate and a process for the preparation thereof is generally known. Specifically the esterification reaction of tetrahydrobenzyl alcohol with (meth)acrylic acid or the transesterification reaction of tetrahydrobenzyl alcohol with a (meth)acrylate ester and successively by the epoxidation reaction with a peracid [Batog, A. E.; Zaitsev, S. Yu.; Kiryushima, N. P.; Zaitseva, V. V. (Inst.Fiz.-Org. Khim. Uglechim., Donetsk, USSR). Zh. Org. Khim, 1982, 18(1), 90-4 (Russ)] are known.

The reaction schemes are represented by the following formulae; 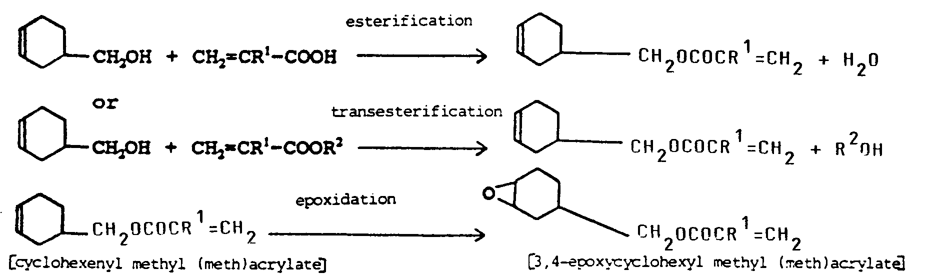 [in the formulae, R¹ is hydrogen or a methyl group, R² is an alkyl group].

However, a process for the preparation of a purified, that is, a commercially useful 3,4-epoxycyclohexyl methyl (meth)acrylate, has not been disclosed up to date.

In particular, there has not been known a process in which even a waste water treatment is taken into consideration or even a small amount of impurity detected by a heptane test described hereinafter can be removed.

On the other hand, it has been known that 3,4-epoxycyclohexyl methyl (meth)acrylate has a disadvantage of exceedingly readily polymerizing, particularly, during the preparation processes, while being stored and/or shipped under the influence of heat, light or other causes.

In order to solve this disadvantage, Japanese Patent Unexamined Publication (Kokai) No. 262,574/1990 teaches a method for preventing polymerization, which uses guinones, etc. together with phosphorous compounds in the presence of molecular state oxygen gas.

However, it has been found by the present inventors that the use of polymerization inhibitors as described hereinabove is not sufficient in the case of commercial preparation processes.

One of the reasons why the effect of this method was not sufficient in that there was not sufficient qualities of 3,4-epoxycyclohexyl methyl (meth)acrylate possessed, since it was not produced on a commercial basis when the Japanese Publication was filed.

Specifically, it is required that low boiling components in a commercially available 3,4-epoxycyclohexyl methyl (meth)acrylate must be removed to the extent of from 2 to 3 %, more preferably, not more than 1 %.

For that purpose, it is required that heating temperatures be raised and/or that processing time be extended during the step of removing the low-boiling ingredients.

However, raising the temperatures or extension of processing time generates, even though in minor amounts, polymers in the product.

It has now been found that the polymers in the product cause problems even though present in minor amounts in commercially available 3,4-epoxycyclohexyl methyl (meth)acrylate.

For example, one of the problems is that the polymers ooze out as adhesive and insoluble substances when preparing intermediate materials of resins for coatings using 3,4-epoxycyclohexyl methyl (meth)acrylate including the polymers. This results in causing various problems in processing and in coatings having a remarkably lower commercial valuation being produced.

It appears that the minor amounts of polymers in the commercially available 3,4-epoxycyclohexyl methyl (meth)acrylate are composed of low molecular weight polymers of 3,4-epoxycyclohexyl methyl (meth)acrylate itself.

The amount of such low molecular weight polymers can be determined in weight % with a measuring method using n-heptane or n-hexane. 10 g of a product is dissolved in 100 cc of n-heptane or n-hexane and the resulting suspension is filtered and weighed (hereinafter, occasionally referred to as HT or HT value in a solubility test).

It is known that a commercially available 3,4-epoxycyclohexyl methyl (meth)acrylate must exhibit an HT value of not more than 0.1 % by weight.

It was found that the method described in Japanese Patent Unexamined Publication (Kokai) No. 262594 / 1990 can only provide 3,4-epoxycyclohexyl methyl (meth)acrylate having a polymer content of more than 0.1 % by weight in HT value, more specifically, 0.14 % by weight or so. These values are not sufficient by a recollected confirmation test using HT carried out thereafter.

That is, further more effective methods for inhibiting polymerizing in each step of the preparation process must be developed in order to produce a purified 3,4-epoxycyclohexyl methyl (meth)acrylate on a commercial basis.

It is noted that 3,4-epoxycyclohexyl methyl (meth)acrylate has an alicyclic epoxy group which tends to exceedingly readily react with an organic acid derived from an organic peracid epoxidation agent. For example, the epoxy group reacts with acetic acid derived from peracetic acid in the case of using peracetic acid as an organic peracid, resulting in polymerization of 3,4-epoxycyclohexyl methyl (meth)acrylate and opening of the epoxy group, particularly in the evaporation step.

Accordingly, it is necessary to remove the organic acid from the crude reaction solution as early as possible in order to maintain a short period of time of contact of the acid with the epoxy group.

More effective methods have not been developed to date.

Furthermore, it is noted that, heretofore, various processes for removing the organic acid and organic peracid have been tried.

In the case of a heat resistant product, a refining process by distillation has usually been carried out (process (a)).

The organic acid or organic peracid which are dissolved in the crude reaction solution can be removed by extraction with water and successively by distillation, in order to prevent the polymerization or the side reaction of an epoxy compound on distilling the crude reaction solution without any refining processes (process (b)).

In cases where the organic acid or organic peracid cannot be removed or where the organic acid in an aqueous solution readily reacts with the epoxy compound, a neutralization process has been usually applied (process (c)).

Furthermore, where the substances which cause the polymerization and the side reaction cannot be removed by merely adjusting the PH of the solution to neutral, the substances are occasionally removed with an aqueous alkali solution.

Distillation is carried out in order to refine after removing the substances by neutralization.

However, the prior art process (a) is often incapable of being applied, because it has a disadvantage in that distillation alone can cause the polymerization or the opening reaction of epoxy groups because of ease of the reaction of epoxy groups with organic acid.

Furthermore, the prior art processes (b) and (c), which are often applied in the case of inability of using the prior art process (a), are also often incapable of being applied in the case of the rapid reaction velocity of epoxy groups with an organic acid.

Still further, the prior art process (c) is often incapable of being used on an industrial basis because of the large amount of product loss and also of the considerable load in water treatments.

As mentioned above, prior art processes (a), (b) and (c) include difficult disadvantages, respectively, in application on an industrial basis.

That is, prior art processes (a), (b) and (c) have been industrially incapable of being used in the cases where an epoxy compound has properties such that a crude reaction solution can not be refined by distillation alone because of polymerization, side reaction and the epoxy group tends to rapidly react with organic acid and/or water.

A first aspect of the present invention relates to a method for reducing the contact time between an organic solution layer and an aqueous solution layer when extracting organic acid and organic peracid with water from a crude reaction solution after the epoxidation reaction.

The first aspect has been found based on the mechanism in which the loss of an epoxidized product is caused by the reaction of the epoxidized product dissolved in water solution with water and organic acid and the resultant concentration reduction of the epoxidized product and further repeatedly resultant dissolving, that is, by the form of a reaction-extraction which accelerates further dissolving into water.

The first aspect is attainable by the use of an apparatus in which the reaction of an epoxy compound with an organic acid and water is reduced by having a shortened contact time between an organic liquid layer and an aqueous liquid layer.

A second aspect of the present invention relates to a method for further removing small amounts of organic acid and organic peracid by extraction with water, and more specifically the method comprises neutralization with alkali after separation from the apparatus as mentioned above.

It is noted that, even though the apparatus as mentioned above can be used to remove almost all of the organic acid and organic peracid by extraction with water, small amounts of them remain.

There is also a disadvantage in the 3,4-epoxycyclohexyl methyl (meth)acrylate product which is refined by distillation in order to remove solvents and other low boiling components such as small amounts of starting materials after removal of the organic peracid by extraction alone with water.

However, the neutralization before extracting the organic acid and organic peracid with water can not be applied on an industrial basis, from the view point of waste water treatments, which is described hereinabove.

The disadvantage in the 3,4-epoxycyclohexyl methyl (meth)acrylate product is low in purity, for example, more specifically less than 90 % in purity, and such unpurified 3,4-epoxycyclohexyl methyl (meth)acrylate undesirably has the tendency of readily polymerizing.

It is noted that it is required that the purity of the commercially available 3,4-epoxycyclohexyl methyl (meth)acrylate be from 94 to 97 % by weight, and that the residual components be primarily starting material, starting solvent and water.

From the above-described viewpoints, and as a result of studies by the present inventors, it has been found that purified 3,4-epoxycyclohexyl methyl(meth)acrylate can be prepared on a commercial basis by various improved steps.

### SUMMARY OF THE INVENTION

An object of the present invention is to provide a purified 3,4-epoxycyclohexyl methyl (meth)acrylate and improved processes for the preparation thereof.

A first aspect of the present invention is an improved process for the preparation of purified 3,4-epoxycyclohexyl methyl (meth)acrylate, characterized in that an apparatus having a short retention time of 1 minute at most is used in extracting with water to remove an organic acid and organic peracid from the crude reaction solution after the epoxidation reaction.

A second aspect of the present invention is an improved process for the preparation of a purified 3,4-epoxycyclohexyl methyl (meth)acrylate, characterized in that organic acid and organic peracid remaining after extraction with water are neutralized with aqueous alkali solution, and then separated.

A third aspect of the present invention is an improved process for the preparation of purified 3,4-epoxycyclohexyl methyl (meth)acrylate, characterized in that 3,4-epoxycyclohexyl methyl (meth)acrylate including low-boiling ingredients is evaporated by different two stage evaporation conditions.

A fourth aspect of the present invention is a process for the preparation of a purified 3,4-epoxycyclohexyl methyl (meth)acrylate, characterized in that a crude reaction solution having 3,4-epoxycyclohexyl methyl (meth)acrylate is processed by the steps of:
(a) extracting organic peracid and organic acid derived from the organic peracid with water using an apparatus having a short retention time to separate an organic solution layer from the aqueous solution layer;
(b) neutralizing the organic solution layer with aqueous alkali solution to form an organic solution layer and an aqueous solution layer, and separating the organic solution layer from the aqueous solution layer;
   and successively
(c) evaporating the organic solution layer at temperatures of not more than 100 °C and at reduced pressures to obtain a 3,4-epoxycyclohexenyl methyl (meth)acrylate solution including low-boiling ingredients of from 3 to 50 % by weight,
   and further
(d) evaporating the 3,4-epoxycyclohexenyl methyl (meth)acrylate solution of (c) at temperatures of not more than 100 °C and at less than 1/2 of the reduced pressures in the above-mentioned step (c) to obtain a purified 3,4-epoxycyclohexenyl methyl (meth) acrylate including the low-boiling ingredients of not more than 1 % by weight .

A fifth aspect of the present invention is a 3,4-epoxycyclohexyl methyl (meth)acrylate having a heptane test value of not more than 0.1 %.

### DETAILED DESCRIPTION OF THE INVENTION

The present invention is described hereinafter in more detail.

According to a first aspect of the present invention, there is provided an improved process for the preparation of 3,4-epoxycyclohexyl methyl (meth)acrylate by the epoxidation reaction of cyclohexenyl methyl (meth)acrylate with an organic peracid, characterized in the use of an apparatus having a short retention time in extracting with water to remove an organic acid and an organic peracid from a crude epoxidation reaction solution.

The crude reaction solution to be used in the first aspect is prepared by the epoxidation reaction of cyclohexenyl methyl (meth)acrylate which is a main starting material, with an organic peracid.

Useful organic peracids include, for example, performic acid, peracetic acid, perpropionic acid, m-chloroperbenzoic acid, trifuluoroperacetic acid and perbenzoic acid.

Of these organic peracids, peracetic acid is the preferred organic peracid, because it is available on an industrial basis at a moderate price and has high stability.

Although the molar ratio of the organic peracid to cyclohexenyl methyl(meth)acrylate, more specifically to the double bond, is theoretically 1/1, the preferred range is from 0.1/1 to 10/1, more preferably from 0.8/1 to 1.5/1.

A ratio of more than 10/1, might be preferred from the view points of the conversion of cyclohexenyl methyl (meth)acrylate to 3,4-epoxycyclohexyl methyl (meth)acrylate, a reduction of the time of period for epoxidizing and a reduction of product losses because of polymerization. However, there are also the disadvantageous results of a side reaction because of the excess of organic peracid or a reduction of selectivity of the organic peracid, and a considerable increase of the recovery cost of the peracid.

On the other hand, if the ratio is not more than 0.1/1, although it is preferred from the view points of a reduction of product losses, a reduction of the side reaction by the organic peracid and an increase of selectivity and further conversion of the organic peracid, there is a considerable increase of the recovery cost of cyclohexenyl methyl (meth)acrylate.

Accordingly, most preferably, a slight excess amount of organic peracid over the theoretical ratio is used because of the decomposition of the organic peracid, even though being small amounts, in the epoxidation reaction.

The epoxidation reaction can be preferably carried out in the presence of a solvent. The use of solvent for dilution is effective for lowering the viscosity of the crude reaction solution and stabilizing the organic peracid, and further lowering the reaction velocity of epoxy group with a resulting organic acid.

The preferred solvents include an aromatic hydrocarbon, such as benzene, toluene, xylene, ethylbenzene, iso-propylbenzene, diethylbenzene, and p-simene, an aliphatic hydrocarbon, such as cyclohexane, n-hexane, heptane, hexane, octane, nonane, decane and decaline, an alcohol, such as cyclohexanol, hexanol, heptanol, octanol, nonanol and furfuryl alcohol, a ketone, such as acetone, methyl ethyl ketone and cyclohexanone, an ester compound, such as ethyl acetate, n-amylacetate, cyclohexyl acetate, isoamyl propionate, and methyl benzoate, a polyvalent alcohol, such as ethylene glycol, propylene glycol, ethylene glycol monomethylether, ethylene glycol monoethylether, ethylene glycol monoethylether acetate, ethylene glycol monomethylether acetate, diethylene glycol monomethylether, diethylene glycol monoethylether or derivatives thereof, a halogenated compound, such as chloroform, dimethyl chloride, carbon tetrachloride, chlorobenzene, and an ether compounds, such as 1,2-dimethoxyetane, etc.

For example, in the case when peracetic acid is used as an organic peracid, ethyl acetate is preferably used as the solvent for dilution.

Although the molar ratio of the solvent to cyclohexenyl methyl (meth)acrylate is preferably from about 0.5/1 to about 5/1, more preferably from about 1.5/1 to about 3/1, if the ratio is less than about 0.5/1, the stabilizing effect to the organic peracid becomes smaller.

On the other hand, even though the ratio is more than about 5/1, the stabilizing effect does not increase so much in comparison with an increase in cost for the recovery of the solvent.

Furthermore, a polymerization inhibitor can be used together with a gas including molecular state oxygen in the case of carrying out the epoxidation reaction.

The preferred polymerization inhibitors include, for example, hydroquinone, hydroquinone monomethylether, p-benzoquinone, cresol, t-butylcatecol, 2,4-dimethyl-6-t-butylphenol, 2-t-butyl-4-methoxyphenol, 3-t-butyl-4-methoxyphenol, 2,6-di-t-butyl-p-cresol, 2,5-dihydroxy-p-quinone, piperidine, ethanolamine, alpha-nitroso-beta-naphthol, diphenylamine, phenothiazine, N-nitrosophenylhydroxylamine and N,N-diethylhydroxylamine, etc.

The amount of the polymerization inhibitor used is preferably from about 0.005 to 5 %, more preferably from about 0.001 to 0.1 % by weight based on cyclohexenyl methyl (meth)acrylate which is the primary starting material.

Still further, a stabilizer for the organic peracid can be optionally used. The preferred stabilizers include, for example, ammonium hydrogen phosphate,
potassium pyrophosphate, sodium phosphate, potassium 2-ethylhexyl pyrophosphate, sodium 2-ethylhexyl pyrophosphate, tripolyphosphoric acid, potassium tripolyphosphate, sodium tripolyphosphate, sodium 2-ethylhexyl pyrophosphate, potassium 2-ethylhexyl pyrophosphate, tetrapolyphosphoric acid, potassium tetrapolyphosphate, sodium tetrapolyphosphate, 2-ethylhexyl tetrapolyphosphate, potassium 2-ethylhexyl tetrapolyphosphate, sodium 2-ethylhexyl tetrapolyphosphate, potassium hexametaphosphate and sodium hexametaphosphate, etc.

The stabilizers can be used alone or in admixtures of more than one stabilizer.

The amount of the stabilizer used for the organic peracid is preferably from about 0.001 to 1 %, more preferably from about 0.01 to 0.2 % by weight based on cyclohexenyl methyl (meth)acrylate which is the primary starting material, in the form of either a powder or a solution with a solvent.

The temperature range of the epoxidation reaction can be usually selected according to the reactivity of the organic peracid, that is, such that the epoxidation reaction advantageously occurs over the decomposition of the organic peracid, and further such that the side reaction such as the opening reaction of the resulting epoxy group with an organic acid derived from the organic peracid does not occur.

In the case of peracetic acid, which is the preferred organic peracid, the preferred temperature range is specifically from about 0 to 70 °C.

If the temperature is lower than about 0 °C, a long time period to complete the epoxidation reaction is required.

On the other hand, if the temperature is higher than about 70 °C, decomposition of peracetic acid occurs.

The epoxidation reaction is usually carried out at ordinary pressure conditions, and also can be optionally carried out at reduced or pressurized conditions.

And, the epoxidation reaction can be carried out by a continuous process or a batchwise process.

In the case of the continuous process, there can preferably be used a piston-flow type process, in the case of the batchwise process, there can be preferably be used, a semi-batch type process in which the organic peracid is successively supplied.

More specifically, the starting material and the solvent are firstly supplied into a reaction vessel, and then the catalyst and the stabilizer are optionally dissolved, and then the organic peracid is successively supplied by dropwise addition as mentioned above.

The completion of the epoxidation reaction is preferably monitored by the concentration of the residual organic peracid or gas chromatography analysis. After the completion of the epoxidation reaction, extraction of the organic acid and organic peracid from the crude reaction solution with water is carried out as described below.

The crude reaction solution generally has a composition composed of 3,4-epoxycyclohexyl methyl (meth)acrylate which is the primary product, small amounts of unreacted cyclohexenyl methyl (meth)acrylate which is the starting material, the residual organic peracid which is used in slightly excess amounts, the organic acid derived from the reacted organic peracid, the optionally used polymerization inhibitor or catalyst, and the solvent.

The crude reaction solution is supplied, as it were, as a starting material in the first aspect of the present invention.

The apparatus used in the first aspect of the present invention essentially has a property of providing a short contact time between the aqueous solution and the organic solution, more specifically, providing a short retention time while passing through the apparatus. The retention time is adjustable depending upon an approvable loss amount of 3,4-epoxycyclohexyl methyl (meth)acrylate which is the primary product and the reaction velocity between the epoxy group and the kind of the organic acid derived from the organic peracid which is used.

A typical example of the apparatus having a short and adjustable retention time is a centrifugal apparatus in which two liquids are capable of counter-currently contacting, which has a rotary body or a drum integrally mounted on a rotary shaft, the rotary body or drum having a plurality of, specifically, 50 or so stages of ring members or perforated cylinders, etc.

Each stage is assembled with mixing chambers and settling chambers in the rotary body or the drum, etc. of the centrifugal apparatus.

A solution to be processed initially containing a solute, and a reagent for extracting circulate counter-currently each other in the rotary body or drum, and mixing and separating operations performed in each stage allow the solute to pass into water.

In the mixing chamber, the two phases are mixed by the high relative speed between the stationary part and rotating wall.

And, in the settling chamber, the two phases previously mixed are separated by centrifugal force.

More specifically, the crude reaction solution and water which is a reagent for extracting are supplied into the rotary body or drum rotating at high speed passing through two pipes connected to a rotating shaft, respectively, and then the solution and water are contacted at the state of a counter-current.

As the result, a heavy solution phase is radially transferred to an outerward direction of the rotary body or drum by the centrifugal force, and a light solution phase is radially transferred to an innerward direction of the shaft.

Effective extraction of the organic acid and the organic peracid from the crude reaction solution can be attained by the counter-currents and transferences at slits between the ring members or holes on perforated cylinders. The organic acid and organic peracid can be extracted with water within a short retention time passing through the apparatus.

Extraction can be carried out within approximately 50 seconds or so even in the case of two solutions having a small difference between specific gravities because of the use of centrifugal force.

Although the period of time required to extract in such apparatuses depends upon the plate efficiency, the numbers of actual plate and the kinds of liquids, that is the specific gravity differences between two liquids, it is generally from about several seconds to approximately 50 seconds or so, and 1 minute at most.

Accordingly, in the case of the present invention, the epoxy group does not react with the organic peracid, the organic acid or water so much, resulting in only minor amounts of product losses.

It is noted that the mechanism and internal structures of such centrifugal type counter-current apparatus are disclosed in US Patent Nos. 3,327,939, 3,814,307, 4,225,079, 4,272,011, 4,326,666 and 4,367,202, etc.

On the other hand, apparatuses such as a mixer-settler type extractor, a ring and plate type extractor and/or a column type extractor, which are generally also used in extraction processes with water, are not appropriate for the present process because of large amounts of product losses by requiring a relatively long retention period of time such as from several minutes to approximately 50 minutes or so.

Furthermore, small amounts of an alkali can optionally be used together with water supplied in the form of counter-current for the purpose of further effectively or completely removing the organic acid and organic peracid in the crude reaction solution.

For example, in the case of using a solvent such as ethyl acetate which has specific gravity less than water, the aqueous alkali solution is inevitably mixed at the zones where the concentrations of the organic acid and organic peracid become lower, more specifically at the slits or the holes in the ring members or the perforated cylinders situated near the shaft.

It is noted that the concentration difference of the organic acid and organic peracid in the crude reaction solution and in aqueous solution act as a driving force for their transference into the aqueous solution.

Accordingly, there is preferably used amounts of alkali corresponding to approximately 1 % of the organic acid and organic peracid in the reaction solution, because of only supplementarily supplying into the zones mentioned above.

Furthermore, it is noted that in the case of having relatively low theoretical plate numbers, the reaction solution can also be passed repeatedly through the apparatus used in the present invention supplying water by counter-current.

However, if a recovery of the organic acid and organic peracid and waste water treatment is not required, the amounts of the alkali used are not limited.

The aqueous solution layer separated in the apparatus in which the organic acid and organic peracid extracted with water are dissolved, can not generally be discharged as waste water without any treatments from the viewpoint of the prevention of environmental polution.

However, for example, a treatment of the solution by a neutralization with alkali can not be applied because of a substantially same process as the neutralization before extraction with water.

Accordingly, when the boiling points of the organic acid and organic peracid are lower than the boiling point of water, they can generally be removed from the solution and they can be recovered with distillation.

On the other hand, when the boiling points of the organic acid and organic peracid are higher than the boiling point of water, the organic acid and organic peracid are removed and recovered by a back extraction method from the solution using a reagent for extracting, more specifically an organic solvent.

Even when the boiling points are higher than the boiling points of water, although distillation can be applied, it can not be applied on an industrial basis because of the considerably large energy costs due to evaporation of water.

The preferred solvents include, for example, benzene, toluene, xylene, ethylbenzene, isopropylbenzene, diethylbenzene, p-cymene, etc. which are aromatic hydrocarbons, cyclohexane, n-hexane, heptane, octane, nonane, decane, decalin, etc. which are aliphatic or alicyclic hydrocarbons, ethyl acetate, n-amyl acetate, cyclohexyl acetate, iso-amyl propionate, methyl ester of benzoic acid which are ester compounds, chloroform, carbon tetrachloride, chlorobenzene, etc. which are halogenated compounds, 1,2-dimethoxyethane, diethyl ether, etc. which are ether compounds.

There can preferably be used solvents having boiling points much lower than the organic acid and organic peracid because of inevitably carrying out distillation after the back extraction.

Furthermore, there can preferably be used solvents having a low solubility in water because of inevitably carrying out waste water treatment.

The water extraction process according to the first aspect of the present invention is preferably carried out at relatively lower temperature conditions from the view point of the lower reactivity of the epoxy group in 3,4-epoxycyclohexyl methyl (meth)acrylate with the organic acid. However, too low temperature conditions occasionally make the property for separating into the two solution layers lower because of an increase of the viscosity and a decrease of the gravity differences.

Accordingly, the preferred temperature range is from about 10 to 30 °C, more preferably from about 15 to 25 °C.

It is noted that the organic acid and also organic peracid should be removed to the extent of less than 0.1 %, preferably less than 0.05 %, respectively, in the reaction solution after extraction in order to sufficiently remove the organic acid and organic peracid in a next process which is an alkali neutralization process and from the view point of reducing the load of waste water treatment.

Although the supplying ratio of water which is a reagent for extracting the organic acid and organic peracid to the crude reaction solution in the extraction process with water is not limited, it is preferably from about 1/2 to 3/1 by weight in order to attain the above-mentioned concentrations of the organic acid and peracid.

According to a second aspect of the present invention, there is provided an improved process for the preparation of a purified 3,4-epoxycyclohexyl methyl (meth)acrylate by the epoxidation reaction of cyclohexenyl methyl (meth)acrylate with an organic peracid, wherein organic acid and organic peracid are removed from a crude reaction solution after the epoxidation reaction by extraction with water and successively neutralized with an aqueous alkali solution.

In the second aspect of the present invention, a solution to be supplied must be the solution after extracting the organic acid and organic peracid with water, which is more specifically, the solution having the organic acid and organic peracid content of less than 0.1 %, more preferably less than 0.05 %.

And, the organic acid and organic peracid must be removed to the extent of less than 0.01 % in the alkali neutralization in order to obtain a purified 3,4-epoxycyclohexyl methyl (meth)acrylate.

Although depending upon the concentrations of the residual organic acid and organic peracid after extraction with water, the concentration of the aqueous alkali is generally from about 0.1 % to 10 %, more preferably from about 1 to 2 %.

If it is more than 10 %, for example, ethyl acetate which is a preferred solvent in the epoxidation reaction occasionally exhibits a tendency toward decomposition.

On the other hand, if it is less than 0.1 %, the organic acid and organic peracid cannot be sufficiently removed.

The preferred temperatures range is from about 0 to 50 °C, preferably from about 10 to 30 °C. In the case of less than 0 °C, it is difficult to separate into two solution layers. On the other hand, in the case of more than 50 °C, organic components tend to be dissolved into the aqueous alkali solution, resulting in increasing the load of waste water treatment.

The neutralization with the aqueous alkali can be carried out by a continuous process or a batchwise process.

In the case of the continuous process, a mixer-settler type apparatus can preferably be used.

It is noted that the above-mentioned centrifugal counter-current apparatus is preferably not used in the alkali neutralization process because of the short retention time, resulting in insufficient removal of the organic acid and peracid.

In the case of the batchwise process, an extracting column type apparatus can preferably be used.

In the alkali neutralization process, the residual (unreacted and remaining in the extraction process with water) organic peracid must be sufficiently removed together with the organic acid, for example, to the extent of not more than 0.01 % by weight based on the reaction solution, for the purpose of a stabilized operation of evaporation in order to remove low-boiling ingredients.

According to a third aspect of the present invention, there is provided an improved process for the preparation of a purified 3,4-epoxycyclohexylmethyl (meth)acrylate by the epoxidation reaction of cyclohexenylmethyl (meth)acrylate with an organic peracid, characterized in that a 3,4-epoxycyclohexyl methyl (meth)acrylate solution including low-boiling ingredients is refined by the step:
(a) evaporating components having low boiling points at temperatures not more than about 100 °C and at reduced pressures to obtain a crude epoxidized solution including low-boiling ingredients of from about 3 to 50% by weight, and successively (sbusequently)
(b) evaporating the low-boiling ingredients at temperatures not more than about 100 °C and at less than 1/2 of the reduced pressures in above-mentioned step (a) to obtain 3,4-epoxycyclohexenyl methyl (meth)acrylate including the low-boiling ingredients of not more than 1 % by weight.

The solution supplied into the first evaporator preferably has a composition composed of 3,4-epoxycyclohexyl methyl (meth)acrylate which is the primary desired product, small amounts of unreacted cyclohexenyl methyl (meth)acrylate which is a starting material, small amounts of the organic peracid and the organic acid unremoved in the extracting process with water and/or in the neutralization process with the aqueous alkali solution, solvent which is a primary low-boiling ingredient to be evaporated, and further small amounts of by-products.

More specifically, the content of the residual organic peracid and organic acid in the solution must be preferably reduced to the extent of less than 100 ppm, respectively.

If the content is more than 100 ppm, the epoxy group in 3,4-epoxycyclohexyl methyl (meth)acrylate reacts with the organic acid under the influence of heating in evaporation, resulting in polymerization of 3,4-epoxycyclohexyl methyl (meth)acrylate and causing suspensions in the heptane test described hereinabove, specifically, the heptane test value of approximately 0.1 % or more.

Although depending upon the kinds of solvents or organic peracids to be used in the epoxidation reaction, the preferred temperatures in evaporation are less than about 100 °C, more specifically from about 40 to 60 °C, from the view point of preventing polymerization of 3,4-epoxycyclohexyl methyl (meth)acrylate.

If the temperatures are more than 100 °C, polymerization of 3,4-epoxycyclohexyl methyl (meth)acrylate and cyclohexenyl methyl(meth)acrylate, even though at small amounts of the organic peracid and organic acid readily tends to occur.

If the temperatures are less than 40 °C, the evaporation velocity is too low.

The evaporations are preferably carried out in a thin-film evaporator.

The reduced pressures range in the first step evaporation is from about 100 to 200 Torr depending upon the kinds of solvents or organic peracids used in the epoxidation reaction, preferably from about 100 to 150 Torr.

And, the reduced pressures range in the second step evaporation is essentially less than about 1/2 of the first step reduced pressure, preferably from about 1/3 to 1/4.

The low-boiling ingredients include solvents, water and minor amounts of the organic acid and organic peracid.

The evaporation process which is the third aspect of the present invention is characterized by being separated into the two stages having different evaporation conditions, and also particularly characterized by controlling the content of the low-boiling ingredients remaining in the first stage evaporation.

The third aspect is based on the mechanism that although initial evaporation of the low-boiling ingredients is relatively easy even though under lowerly reduced pressure conditions, final evaporation is relatively difficult without highly reduced pressure conditions.

That is, the contents of the low-boiling ingredients are essentially maintained in the range of from about 3 to 50 % by weight based on the total weight of the crude reaction solution, preferably from about 5 to 20 % by weight, more preferably about 10 % or so.

If the contents are less than about 3 % by weight, highly reduced pressure in the first stage evaporation is required and the evaporated solvents can not be economically recovered without many losses in the case of catching by a condenser.

On the other hand, if the contents are more than about 50 % by weight, solvents can not be economically recovered without many losses in the case of catching by a condenser because of the highly reduced pressures in the second stage.

The content of the low-boiling ingredients in the product under the second stage evaporation are essentially not more than about 1 % by weight.

If the contents are more than about 1 %, the product primarily including 3,4-epoxycyclohexyl methyl (meth)acrylate is not commercially available.

According to a fourth aspect of the present invention, there is provided a process for the preparation of a purified 3,4-epoxycyclohexenyl methyl (meth)acrylate from a crude reaction solution obtained by the epoxidation reaction of cyclohexenyl methyl (meth)acrylate with an organic peracid, characterized by the step:
(a) extracting said organic peracid and an organic acid derived from said organic peracid with water using an apparatus having a short retention time to separate an organic solution layer from an aqueous solution layer,
(b) neutralizing organic solution layer with an aqueous alkali solution to form an organic solution layer and an aqueous solution layer, separating said organic solution layer from said aqueous solution layer,
   and successively
(c) evaporating said organic solution layer at temperatures not more than 100 °C and at reduced pressures to obtain a 3,4-epoxycyclohexenyl methyl (meth)acrylate solution including low-boiling ingredients of from about 3 to 50 % by weight,
   and further
(d) evaporating said 3,4-epoxycyclohexenyl methyl (meth)acrylate solution at temperatures not more than about 100 °C and at less than 1/2 of the reduced pressures in the above-mentioned (c) to obtain a purified 3,4-epoxycyclohexenyl methyl (meth)acrylate including the low-boiling ingredients of not more than 1 % by weight.

The fourth aspect is composed of a series of the combination of the above-mentioned first aspect, second aspect and third aspect.

The 3,4-epoxycyclohexyl methyl (meth)acrylate as prepared according to the present invention can be used in a composition including from about 0.01 to 0.1 parts by weight of an organic phosphorous compound represented by general formulae (I) and/or (II) described hereinafter; 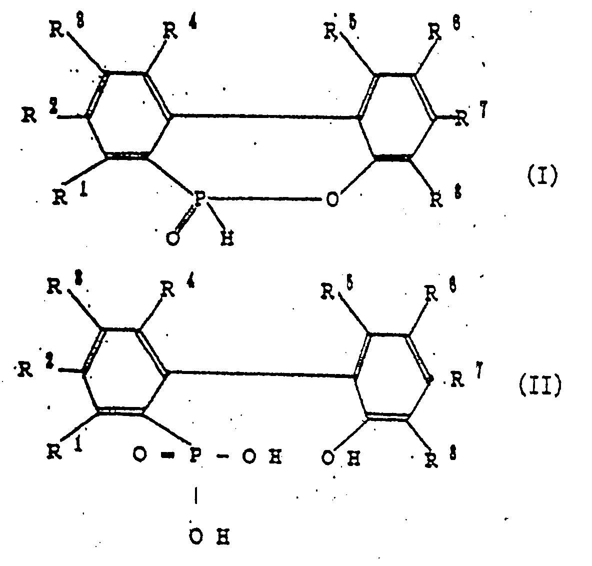 [in the formulae (I) and (II), R¹ to R⁸ are each independently hydrogen, a halogen, and/or a monovalent aliphatic or aromatic substituent group having from 1 to 10 carbons.

The organic phosphorous compounds are used for the purpose of preventing coloring of 3,4-epoxycyclohexyl methyl (meth)acrylate.

As described previously, it is known that 3,4-epoxycyclohexyl methyl (meth)acrylate is exceedingly readily colored in the preparation processes, while being stored and/or shipped because of oxidation by air or other causes. For example, it appears as a possible mechanism of coloring that 3,4-epoxycyclohexyl methyl (meth)acrylate itself polymerizes or by-reacts, even though in very minor amounts, because of its high reactivity.

The preferred color hue value in a commercially available 3,4-epoxycyclohexyl methyl (meth)acrylate is usually less than about 200 in APHA value, if taking it into consideration of the various uses, desirably less than about 100.

It may be generally considered that purification of 3,4-epoxycyclohexyl methyl (meth)acrylate by distillation is a suitable method for preventing coloring.

However, it is not preferable due to history of heating even though under reduced pressures.

Accordingly, it appears that a chemical treatment or an absorption treatment instead of distillation is more preferable.

In the chemical treatment, for example, there have been added molecular state oxygen, an oxidant such as hydrogen peroxide, a reducing agent such as sodium borohydride, sodium hydrogenated bis(2-methoxyethoxy)aluminum, a polymerization inhibitor such as hydroquinone, an antioxidant such as BHT (2,6-ditertbutyl-4-methyl-phenol), BHA (butyl hydroxy anisole), a blocking agent for metal such as EDTA, trioctyl phthalate, etc. in the preparation processes.

In the absorption treatment, for example, it is known that there have been used activated carbon which is a conventional absorbent, an activated clay, zeolite, a highly porous polymer, etc.

However, there are only very minor effects for preventing coloring or for discoloring according to such conventional chemical treatment or absorption treatment.

Furthermore, Japanese Patent Application No. 191267/1990 discloses the use of a hydrotalcite compound for removing coloring components in 3,4-epoxycyclohexyl methyl (meth)acrylate.

However, their coloring occurs again after a long time of period in the use of the compound.

The essential organic phosphorous compounds include 3,4,5,6-dibenzo-1,2-oxaphosphane-2-oxide, 9,10-dihydro-9-oxa-10-phosphaphenanthrene-10-oxide (for example, HCA which is a trade name, manufactured by Sanko Chemical, Ltd.), 6,8-dichloro-9,10-dihydro-9-oxa-10-phosphaphenanthrene-10-oxide, 6,8-di(tert-butyl)-9,10-dihydro-9-oxa-10-phosphaphenanthrene-10-oxide, etc., and further a hydrated compound thereof.

The organic phosphorous compounds which are used in the above mentioned composition are represented by formula (I); 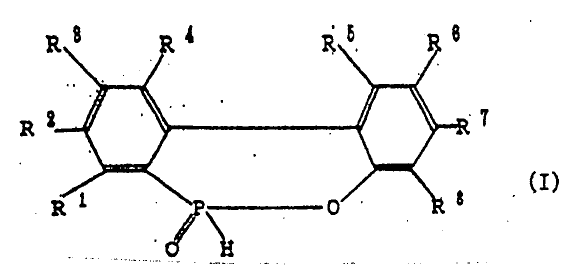 , and the hydrated compound are represented by formula (II); 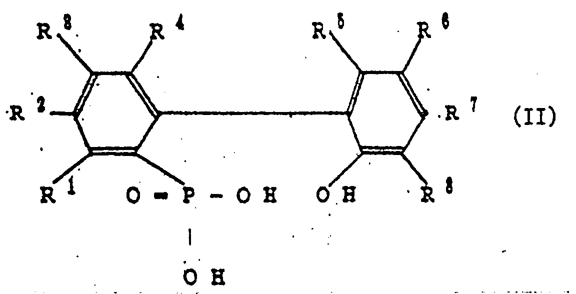 [in the formulae (I) and (II), R¹ to R⁸ are each independently hydrogen, halogen, and/or a monovalent aliphatic or aromatic substituent group having brom 1 to 10 carbons.

The organic phosphorous compounds can be added either in the form of powder or a solution with a solvent.

Although the organic phosphorous compounds may also be added even in any step of the present preparation process, it is preferably mixed in the final product.

The amounts of the compound represented by formulae (I) and (II) are essentially from about 0.001 to 1 parts, preferably from about 0.01 to 0.2 parts by weight.

If the amounts used are less than 0.001 parts by weight, the effect as a polymerization inhibitor is small.

On the other hand, if the amounts used are more than 1 part by weight, an adverse affection is casued instead of the effect as a polymerization inhibitor.

According to a fifth aspect of the present invention, there is provided 3,4-epoxycyclohexyl methyl (meth)acrylate having a heptane test value of less than 0.1 % by weight.

The 3,4-epoxycyclohexyl methyl (meth)acrylate having a heptane test value of less than 0.1 % by weight can be prepared by the combined processes of the above-mentioned first aspect, second aspect and third aspect, and/or fourth aspect alone.

The heptane test value relating to a conventional 3,4-epoxycyclohexyl methyl (meth)acrylate has been more than 0.1 % by weight, more specifically approximately 0.14 % by weight or so.

The heptane test value corresponds to the amounts of polymers having a low molecular weight composed of 3,4-epoxycyclohexyl methyl (meth)acrylate itself.

The contents of the polymers having a low molecular weight can be shown by weight % with a measuring method using n-heptane or n-hexane, in which 10 g of a product is dissolved in 100 cc of n-heptane or n-hexane and resulting suspensions are filtered and weighed.

3,4-epoxycyclohexyl methyl (meth)acrylate prepared by the combined processes of the above-mentioned first aspect, second aspect and third aspect, and/or fourth aspect alone exhibits usually the heptane test value of 0.02 % by weight or so.

The following, Synthesis Examples, Examples and Comparative Examples are provided in order to more specifically illustrate the present nvention.

### [Synthesis Example 1--- Preparation of a crude reaction solution including 3,4-epoxycyclohexyl methyl methacrylate]

A SUS 316-made reaction vessel having a capacity of 15 liter equipped with a stirrer and a jacket for cooling was charged with 3913 parts of 3-cyclohexenyl methyl (meth)acrylate (hereinafter, referred to as CHMA), 7826 parts of ethyl acetate, 5.85 parts of hydroquinone monomethylether which is a polymerization inhibitor and 2.35 parts of sodium tripolyphosphate which is a stabilizer for peracid, followed by raising the internal temperature to 45 °C.

Successively, 6329 parts of ethyl acetate solution including 30 % of peracetic acid was added dropwise over 4 hours, followed by aging for 2 hours. The internal temperature was maintained at 50 °C while adding dropwise and aging, to obtain 18076 parts of a crude reaction solution including 3,4-epoxycyclohexyl methyl methacrylate (hereinafter, referred to as METHB).

### [Example 1 --- a water extraction test with a centrifugal extractor]

The crude reaction solution obtained in Synthesis Example 1 was introduced from an inlet for a light solution phase at 2108 g/minute and water was introduced from an inlet for a heavy solution phase at 3590 g/minute into a counter-current type centrifugal extractor equipped with a rotor having an outer diameter of 46 cm and an inner diameter of 25 mm which rotates at 4000 r.p.m.

And, a light solution layer was obtained at 1664 g/minute from an outlet for the light solution layer and a heavy solution layer was obtained at 4034 g/minute from an outlet for the heavy solution layer.

The light solution layer was introduced again from the inlet for the light solution layer at 2108 g/minute and water was introduced from the inlet for the heavy solution layer at 3590 g/minute.

And, a light solution layer was obtained at 1877 g/minute from the outlet for the light solution layer and a heavy solution layer was obtained at 3821 g/minute from the outlet for the heavy solution layer.

The concentrations of acetic acid and peracetic acid in the crude reaction solution before extracting with water were 10.66 % and 1.15 %, respectively.

The concentrations of acetic acid and peracetic acid in the light solution layer after extracting twice with water were 400 ppm and 150 ppm, respectively.

99 % of METHB dissolved in the crude reaction solution was recovered in the light solution layer after extracting twice with water.

### [Comparative Example 1 --- a water extraction test supposing a column type extractor]

A flask having a capacity of 2 liter equipped with a stirrer was charged with 1000 parts of a crude reaction solution obtained by the same procedures as described in Synthesis Example 1 and 1000 parts of water, followed by agitating for 60 minutes at 20 °C and settling for 30 minutes.

As the result, approximately 5 % of METHB dissolved in the crude reaction solution disappeared under the influence of the reaction of METHB with an aqueous acetic acid solution.

### [Synthesis Example 2 --- Preparation of a crude reaction solution including 3,4-epoxycyclohexyl methyl acrylate]

A SUS 316-made reaction vessel having a capacity of 20 liter equipped with a stirrer and a jacket for cooling was charged with 3000 parts of 3-cyclohexenyl methyl acrylate (hereinafter, referred to as CHAA), 11100 parts of ethyl acetate, 0.9 part of hydroquinone monomethylether which is a polymerization inhibitor and 9.0 parts of sodium tripolyphosphate, followed by introducing a mixed gas composed of oxygen and nitrogen (oxygen/nitrogen = 10/90 by volume) at 32 normal liter/hour from a tube and raising the internal temperature to 40 °C.

Successively, 5623 parts of ethyl acetate solution including 30 % of peracetic acid was added dropwise over 4 hours with a pump maintaining the reaction temperature at 40 °C, followed by aging for 5 hours after the addition of peracetic acid to complete the epoxidation reaction and to obtain 19723 parts of a crude reaction solution including 3,4-epoxycyclohexyl methyl acrylate (hereinafter, referred to as AETHB).

### [Example 2 --- a water extraction test with a centrifugal extractor]

The crude reaction solution obtained in Synthesis Example 2 was introduced from an inlet for a light solution phase at 2108 g/minute and water was introduced from an inlet for a heavy solution phase at 3621 g/minute into a counter-current type centrifugal extractor equipped with a rotor having an outer diameter of 46 cm and an inner diameter of 25 mm which rotates at 4000 r.p.m.

And, a light solution layer was obtained at 1713 g/minute from an outlet for the light solution phase and a heavy solution layer was obtained at 4016 g/minute from an outlet for the heavy solution phase.

The light solution layer was introduced again from the inlet for the light solution phase at 2108 g/minute and water was introduced from the inlet for the heavy solution phase at 3590 g/minute.

And, a light solution layer was obtained at 1865 g/minute from the outlet for the light solution phase and a heavy solution layer was obtained at 3833 g/minute from the outlet for the heavy solution phase.

The concentrations of acetic acid and peracetic acid in the crude reaction solution before extracting with water were 8.96 % and 1.51 %, respectively.

The concentrations of acetic acid and peracetic acid in the light solution layer after extracting twice with water were 400 ppm and 150 ppm, respectively.

99 % of AETHB dissolved in the crude reaction solution was recovered in the light solution layer after extracting in twice with water.

### [Example 3 --- an alkali neutralization test]

A SUS 316-made vessel for mixing equipped with a stirrer and a jacket for cooling having a capacity of 15 liter was charged with 3000 parts of the light solution layer obtained in Example 2, successively, 3000 parts of an aqueous solution including 1 % of NaOH was charged into the vessel, followed by agitating for 1 hour maintaining at the temperature of 10 °C.

After settling for 30 minutes, 2790 parts of an upper solution layer was separated from a lower solution layer.

The concentrations of acetic acid and peracetic acid in the upper solution layer were less than 100 ppm, respectively.

### [Example 4 --- an alkali neutralization test]

The same procedures as described in Example 3 were repeated, except that there was charged 3000 parts of an aqueous solution including 0.5 % of NaOH.

The concentrations of acetic acid and peracetic acid in the upper solution layer were less than 100 ppm, respectively.

### [Example 5 --- an alkali neutralization test]

The same procedures as described in Example 5 were repeated, except that there was charged 3000 parts of an aqueous solution including 0.1 % of NaOH.

The concentrations of acetic acid and peracetic acid in the upper solution layer were less than 100 ppm, respectively.

### [Example 6 --- an alkali neutralization test]

The same procedures as described in Example 3 were repeated, except that there was charged 300 parts of an aqueous solution including 1 % of NaOH. The concentrations of acetic acid and peracetic acid in the upper solution layer were less than 100 ppm, respectively.

### [Comparative Example 2 --- a water extraction test supposing a column type extractor]

A SUS316-made mixing vessel having a capacity of 15 liter equipped with a stirrer was charged with 3000 parts of a crude reaction solution obtained by the same procedures as described in Synthesis Example 2 and 3000 parts of a distilled water, followed by agitating for 60 minutes at 10 °C and settling for 60 minutes, resulting in two solution layers.

2790 parts of the light solution layer was separated from the heavy solution layer.

As the result, approximately 5 % of AETHB dissolved in the crude reaction solution disappeared under the influence of the reaction of AETHB with an aqueous acetic acid.

### [Comparative Example 3 --- an evaporation test after a water extraction test supposing a column type extractor and without neutralization with an aqueous alkali solution]

0.21 part of hydroquinone monomethylether was added in 2790 parts of the upper solution layer obtained in Comparative Example 2, the solution was supplied into a SUS-made Smith falling film type evaporator maintained at the temperature of 60 °C and the reduced pressure of 150 mm Hg supplying a mixed gas composed of oxygen and nitrogen at 32 liter/hour from the bottom of the evaporator, to which a line for discharging a liquid is connected, to obtain a solution including 5 % of low boiling components.

Successively, the solution discharged from the bottom was supplied again into the SUS-made Smith falling film type evaporator at the temperature of 60 °C and the reduced pressure of 40 mm Hg supplying a mixed gas composed of oxygen and nitrogen at 32 liter/hour from the bottom of the evaporator to which a line for discharging a liquid is connected, to obtain 538 parts of a product including not more than 1 % of low boiling ingredients.

The concentration of AETHB in the product was 86.5 % by a gas chromatography analysis.

### [Example 7 --- an evaporation test]

0.21 part of hydroquinone monomethylether was added in 2790 parts of the upper solution layer obtained in Example 3, the solution was supplied into a SUS-made Smith falling film type evaporator maintained at the temperature of 60 °C and the reduced pressure of 150 mm Hg supplying a mixed gas composed of oxygen and nitrogen at 32 liter/hour from the bottom of the evaporator, to which a line for discharging a liquid is connected, to obtain a solution including 5 % of low-boiling ingredients.

Successively, the solution discharged from the bottom was supplied again into the SUS-made Smith falling film type evaporator at the temperature of 60 °C and the reduced pressure of 40 mm Hg supplying a mixed gas composed of oxygen and nitrogen at 32 liter/hour from the bottom of the evaporator to which a line for discharging a liquid is connected, to obtain 538 parts of a product including not more than 1 % of low-boiling ingredients.

The concentration of AETHB in the product was 96.4 % by a gas chromatography analysis.

The heptane test value of the product was 0.02 %.

### [Example 8 --- an evaporation test]

The same procedures as described in Example 7 were repeated, except that the upper solution layer obtained in Example 4 was used to obtain 538 parts of a product. The concentration of AETHB in the product was 95.7 % by a gas chromatography analysis.

The heptane test value of the product was 0.02 %.

### [Comparative Example 4 --- an evaporation test without neutralization with alkali after extracting with water]

0.21 part of hydroquinone monomethylether was added in 2300 parts of the same upper solution layer as obtained in Example 1, the solution was supplied into a SUS-made Smith falling film type evaporator maintained at the temperature of 60 °C and the reduced pressure of 150 mm Hg supplying a mixed gas composed of oxygen and nitrogen at 32 liter/hour from the bottom of the evaporator, to which a line for discharging a liquid is connected, to obtain a solution including 5 % of low-boiling ingredients.

Successively, the solution discharged from the bottom was supplied again into the SUS-made Smith falling film type evaporator at the temperature of 60 °C and the reduced pressure of 40 mm Hg supplying a mixed gas composed of oxygen and nitrogen at 32 liter/hour from the bottom of the evaporator to which a line for discharging a liquid is connected, to obtain a product including not more than 1 % of low-boiling ingredients.

The concentration of METHB in the product was 89.5 % by a gas chromatography analysis.

### [Example 9 --- a coloring test after mixing an organic phosphorous compound]

0.1 part of 9,10-dihydro-9-oxa-10-phosphaphenanthrene-10-oxide was mixed with 100 parts of the product which has a color hue of 40 in APHA value obtained in Example 7.

The mixture was stored for three months at 30 °C. As the result, the APHA value changed to 50.

### [Comparative Example 4 --- a coloring test without mixing an organic phosphorous compound]

The product which has a color hue of 40 in APHA value obtained in Example 7 without mixing any additives was stored for three months at 30 °C. As the result, the APHA value changed to 400.

### [Comparative Example 5 --- a coloring test after mixing large amounts of an organic phosphorous compound]

1 part of 9,10-dihydro-9-oxa-10-phosphaphenanthrene-10-oxide was mixed with 100 parts of the product which has AETHB contents of 96.4 obtained in Example 7. The mixture was stored for three months at 30 °C. As the result, the AETHB contents changed to 91.2 %.

### [Comparative Example 6 --- a coloring test after mixing small amounts of an organic phosphorous compound]

0.001 part of 9,10-dihydro-9-oxa-10-phosphaphenanthrene-10-oxide was mixed with 100 parts of the product which has a color hue of 40 in APHA value obtained in Example 7. The mixture was stored for three months at 30 °C. As the result, the APHA value changed to 130.

### [Comparative Example 7 --- a coloring test after mixing of a conventional coloring inhibitor]

0.1 part of BHT which is an antioxidant was mixed with 100 parts of the product which has a color hue of 40 in APHA value obtained in Example 7. The mixture was stored for three months at 30 °C. As the result, the APHA value changed to 150.

### [Comparative Example 8 --- a coloring test after mixing of a conventional coloring inhibitor]

0.1 part of BHA which is an antioxidant was mixed with 100 parts of the product which has a color hue of 40 in APHA value obtained in Example 7. The mixture was stored for three months at 30 °C. As the result, the APHA value changed to 180.

### [Comparative Example 9 --- a coloring test after mixing of a conventional coloring inhibitor]

0.1 part of Irganox (manufactured by Ciba-Geigy Corp.) which is an antioxidant was mixed with 100 parts of the product which has a color hue of 40 in APHA value obtained in Example 7.

The mixture was stored for three months at 30 °C. As the result, the APHA value changed to 160.

### [Comparative Example 10 --- a coloring test after mixing of a conventional coloring inhibitor]

0.1 part of Sanol (manufactured by Ciba-Geigy Corp.) which is an antioxidant was fixed with 100 parts of the product which has a color hue of 40 in APHA value obtained in Example 7.

The mixture was stored for three months at 30 °C. As the result, the APHA value changed to 350.

### [Comparative Example 11 --- an evaporation test after alkali neutralization treatments thrice without extraction with water]

3000 parts of a crude reaction solution obtained by the same procedures as described in Synthesis Example 2 was mixed with 2500 parts of an aqueous solution including 10 % of NaOH, followed by stirring for 30 minutes and settling for 30 minutes, and then followed by separating the reaction solution.

The separated solution was mixed again with 2500 parts of an aqueous solution including 10 % of NaOH, followed by stirring for 30 minutes and settling for 30 minutes, and then followed by separating the reaction solution.

The contents of peracetic acid in the separated solution were 0.02 %, and acetic acid was completely removed.

The separated solution was further mixed with 2500 parts of an aqueous solution including 1 % of NaOH, followed by stirring for 30 minutes and settling for 30 minutes, and then followed by separating the reaction solution. The contents of peracetic acid in the separated solution were less than 100 ppm.

The same procedures as described in Example 7 were repeated, except that the solution obtained in the alkali neutralization treatments was used to obtain a product.

The concentration of AETHB in the product was 94.5 % by a gas chromatography analysis. And, the contents of ethyl acetate, CHAA and other components were 1.8 %, 1.0 % and 2.7 %, respectively.

The heptane test value of the product was 0.25 %, despite of evaporation in peracetic acid contents of less than 100 ppm and complete removal of acetic acid, because of a long contacting time under the coexistence of peracetic acid, acetic acid and water.

While the invention has been described in detail and with reference to specific embodiments thereof, it will be apparent to one skilled in the art that various changes and modifications can be made therein without departing from the spirit and scope thereof.

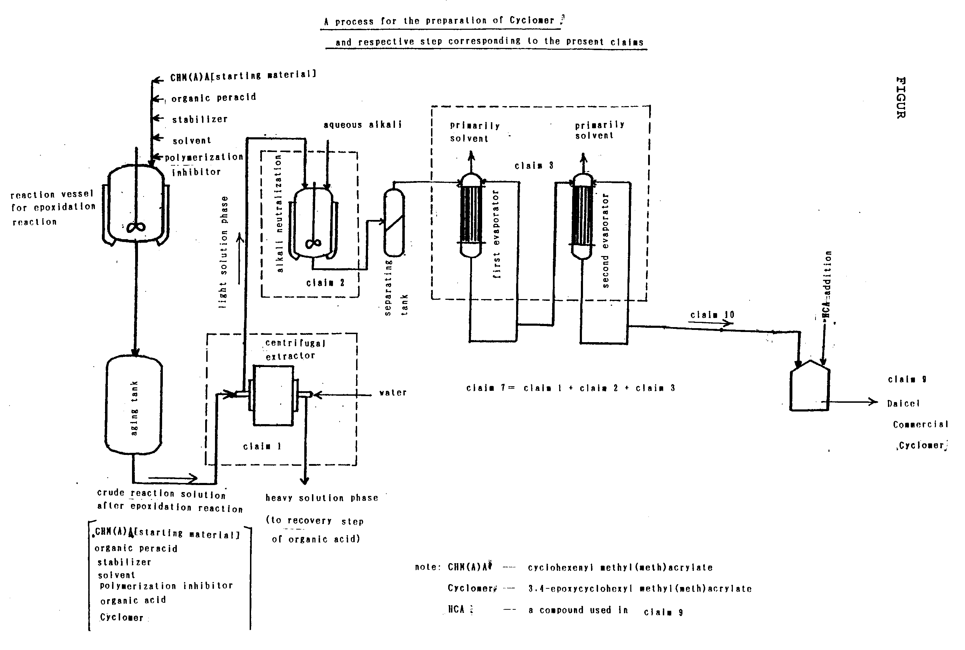

## Claims

1. A process for the preparation of 3,4-epoxycyclohexyl methyl (meth)acrylate by an epoxidation reaction of cyclohexenyl methyl (meth)acrylate with an organic peracid, characterized in that an apparatus having a short retention time of 1 minute at most is used in extracting with water to remove organic peracid and organic acid derived from the organic peracid from a crude epoxidation reaction solution.

2. A process for the preparation of 3,4-epoxycyclohexyl methyl (meth)acrylate by an epoxidation reaction of cyclohexenyl methyl (meth)acrylate with an organic peracid, characterized in that the organic peracid and organic acid derived from the organic peracid are removed from a crude epoxidation reaction solution by extracting with water and successive neutralization with an aqueous alkali solution, and further said crude epoxidation reaction solution is separated by static gravity.

3. A process for the preparation of 3,4-epoxycyclohexyl methyl (meth)acrylate by an epoxidation reaction of cyclohexenyl methyl (meth)acrylate with an organic peracid, characterized in that a 3,4-epoxycyclohexyl methyl (meth)acrylate solution including low-boiling ingredients is refined by the steps of:
(a) evaporating the low-boiling ingredients at temperatures not more than about 100 °C and at reduced pressures to obtain a crude epoxidized solution including the low-boiling ingredients of from about 3 to 50 % by weight, and successively
(b) evaporating the low-boiling ingredients at temperatures not more than about 100 °C and at less than 1/2 of the reduced pressures used in step (a) to obtain 3,4-epoxycyclohexenyl methyl (meth)acrylate including the low-boiling ingredients of not more than 1 % by weight.

4. A process according to claims 1 to 3, wherein said organic peracid is peracetic acid and said organic acid is acetic acid.

5. A process according to claim 1, wherein said apparatus is a centrifugal extractor.

6. A process accodring to claim 3, wherein said 3,4-epoxycyclohexenyl methyl (meth)acrylate solution includes an organic peracid and organic acid of less than 100 ppm, respectively.

7. A process for the preparation of 3,4-epoxycyclohexenyl methyl (meth)acrylate from a crude reaction solution obtained by the epoxidation reaction of cyclohexenyl methyl (meth)acrylate with an organic peracid, comprising:
(a) extracting said organic peracid and organic acid derived from said organic peracid with water using an apparatus having a short retention time to separate an organic solution layer from an aqueous solution layer,
(b) neutralizing said organic solution layer with an aqueous alkali solution to form an organic solution layer and an aqueous solution layer, and separating said organic solution layer from said aqueous solution layer,
and successively
(c) evaporating said organic solution layer at temperatures not more than 100 °C and at reduced pressures to obtain a 3,4-epoxycyclohexenyl methyl (meth)acrylate solution including low-boiling ingredients of from 3 to 50 % by weight,
and further
(d) evaporating said 3,4-epoxycyclohexenyl methyl (meth)acrylate solution at temperatures not more than 100 °C and at less than 1/2 of the reduced pressures in step (c) to obtain a purified 3,4-epoxycyclohexen yl methyl (meth)acrylate including the low-boiling ingredients of not more than 1 % by weight.

8. A process according to claim 7, wherein said organic peracid is peracetic acid and said organic acid is acetic acid.

9. 3,4-epoxycyclohexyl methyl (meth)acrylate having a heptane test value of less than 0.1 %.

## Patentansprüche

1. Verfahren zur Herstellung von 3,4-Epoxycyclohexylmethyl(meth)acrylat durch Epoxidierung von Cyclohexenylmethyl(meth)acrylat mit einer organischen Persäure, dadurch gekennzeichnet, daß zur Extraktion mit Wasser zur Entfernung der organischen Persäure und der von der organischen Persäure abgeleiteten organischen Säure aus der rohen Epoxidierungslösung eine Vorrichtung mit einer kurzen Verweilzeit von höchstens einer Minute verwendet wird.

2. Verfahren zur Herstellung von 3,4-Epoxycyclohexylmethyl(meth)acrylat durch Epoxidierung von Cyclohexenylmethyl(meth)acrylat mit einer organischen Persäure, dadurch gekennzeichnet, daß die organische Persäure und die von der organischen Persäure abgeleitete organische Säure aus der rohen Epoxidierungslösung durch Extraktion mit Wasser und nachfolgende Neutralisation mit einer wässerigen Alkalilösung entfernt werden, und die rohe Epoxidierungslösung durch statische Schwerkraft abgetrennt wird.

3. Verfahren zur Herstellung von 3,4-Epoxycyclohexylmethyl(meth)acrylat durch Epoxidierung von Cyclohexenylmethyl(meth)acrylat mit einer organischen Persäure, dadurch gekennzeichnet, daß eine 3,4-Epoxycyclohexylmethyl(meth)acrylatlösung, die niedrigsiedende Bestandteile enthält, mittels der Stufen raffiniert wird:
(a) Verdampfen der niedrigsiedenden Bestandteile bei Temperaturen, die nicht größer als ca. 100 °C sind, und bei einem vermindertem Druck, um eine rohe epoxidierte Lösung zu erhalten, die die niedrigsiedenden Bestandteile in einer Menge von ca. 3 bis 50 Gew.-% enthält, und nachfolgendes
(b) Verdampfen der niedrigsiedenden Bestandteile bei Temperaturen, die nicht größer als 100 °C sind, und bei weniger als der Hälfte des in Stufe (a) verwendeten verminderten Drucks, um ein 3,4-Epoxycyclohexenylmethyl-(meth)acrylat zu erhalten, das die niedrigsiedenden Bestandteile in einer Menge von nicht mehr als 1 Gew.-% enthält.

4. Verfahren nach einem der Ansprüche 1 bis 3, dadurch gekennzeichnet, daß die organische Persäure Peressigsäure und die organische Säure Essigsäure ist.

5. Verfahren nach Anspruch 1, dadurch gekennzeichnet, daß die Vorrichtung ein Zentrifugalextraktor ist

6. Verfahren nach Anspruch 3, dadurch gekennzeichnet, daß die 3,4-Epoxycyclohexenylmethyl(meth)acrylatlösung die organische Persäure bzw. organische Säure in einer Menge von weniger als 100 ppm enthält.

7. Verfahren zur Herstellung von 3,4-Epoxycyclohexenylmethyl(meth)acrylat aus einer rohen Reaktionslösung, die durch Epoxidierung von Cyclohexenylmethyl(meth)acrylat mit einer organischen Persäure erhalten wurde, dadurch gekennzeichnet, daß es umfaßt:
(a) Extrahieren der organischen Persäure und der von dieser organischen Persäure abgeleiteten organischen Säure mit Wasser unter Verwendung einer Vorrichtung mit einer kurzen Verweilzeit, um eine organische Lösungsschicht von einer wässerigen Lösungsschicht abzutrennen,
(b) Neutralisieren der organischen Lösungsschicht mit einer wässerigen Alkalilösung, um eine organische Lösungsschicht und eine wässerige Lösungsschicht auszubilden, und Abtrennen der organischen Lösungsschicht von der wässerigen Lösungsschicht, und nachfolgendes
(c) Verdampfen der organischen Lösungsschicht bei Temperaturen von nicht mehr als 100 °C und bei einem vermindertem Druck, um eine 3,4-Epoxycyclohexenylmethyl-(meth)acrylatlösung zu erhalten, die niedrigsiedende Bestandteile in einer Menge von 3 bis 50 Gew.-% enthält, und dann
(d) Verdampfen der 3,4-Epoxycyclohexenylmethyl(meth)-acrylatlösung bei Temperaturen von nicht mehr als 100 °C und bei weniger als der Hälfte des in Stufe (c) verwendeten verminderten Druckes, um ein gereinigtes 3,4-Epoxycyclohexenylmethyl(meth)acrylat zu erhalten, das die niedrigsiedenden Bestandteile in einer Menge von nicht mehr als 1 Gew.-% enthält.

8. Verfahren nach Anspruch 7, dadurch gekennzeichnet, daß die organische Persäure Peressigsäure und die organische Säure Essigsäure ist.

9. 3,4-Epoxycyclohexylmethyl(meth)acrylat mit einem Heptantestwert von weniger als 0,1 %.

## Revendications

1. Procédé de préparation du (méth)acrylate de 3,4-époxycyclohexylméthyle au moyen d'une réaction d'époxydation du (meth)acrylate de cyclohexènylméthyle avec un peracide organique, caractérisé en ce qu'un appareil ayant un temps de rétention court de 1 minute au maximum est utilisé pour une extraction à l'eau afin d'extraire le peracide organique et l'acide organique dérivé du peracide organique d'une solution réactionnelle d'époxydation brute.

2. Procédé de préparation du (méth)acrylate de 3,4-époxycyclohexylméthyle au moyen d'une réaction d'époxydation du (meth)acrylate de cyclohexènylméthyle avec un peracide organique, caractérisé en ce que le peracide organique et l'acide organique dérivé du peracide organique sont éliminés d'une solution réactionnelle d'époxydation brute par une extraction à l'eau suivie d'une neutralisation par une solution aqueuse alcaline, et ensuite ladite solution réactionnelle d'époxydation brute est séparée par gravité statique.

3. Procédé de préparation du (méth)acrylate de 3,4-époxycyclohexylméthyle au moyen d'une réaction d'époxydation du (meth)acrylate de cyclohexènylméthyle avec un peracide organique, caractérisé en ce que une solution de (méth)acrylate de 3,4-époxycyclohexylméthyle comprenant des ingrédients à bas point d'ébullition est purifiée au moyen des étapes consistant à :
(a) évaporer les ingrédients à bas point d'ébullition à des températures n'étant pas supérieures à environ 100°C et à des pressions réduites pour obtenir une solution époxydée brute comprenant les ingrédients à bas point d'ébullition dans une proportion allant de environ 3 à 50% en poids, et à la suite
(b) évaporer les ingrédients à bas point d'ébullition à des températures n'étant pas supérieures à environ 100°C et à une pression inférieure à la moitié des pressions réduites utilisées lors de l'étape (a) pour obtenir le (méth)acrylate de 3,4-époxycyclohexénylméthyle comprenant les ingrédients à bas point d'ébullition dans une proportion n'étant pas supérieure à 1% en poids.

4. Procédé selon les revendications 1 à 3, dans lequel ledit peracide organique est l'acide peracétique et ledit acide organique est l'acide acétique.

5. Procédé selon la revendication 1, dans lequel ledit appareil est un extracteur centrifuge.

6. Procédé selon la revendication 3, dans lequel ladite solution (méth)acrylate de 3,4-époxycyclohexènylméthyle comprend un peracide organique et un acide organique dans une proportion inférieure à 100 ppm, respectivement.

7. Procédé de préparation du (méth)acrylate de 3,4-époxycyclohexènylméthyle à partir d'une solution réactionnelle brute obtenue au moyen d'une réaction d'époxydation du (méth)acrylate de cyclohexènylméthyle avec un peracide organique, comprenant :
(a) l'extraction dudit peracide organique et dudit acide organique dérivé dudit peracide organique par de l'eau en utilisant un appreil présentant un temps de rétention court pour séparer une couche de solution organique d'une couche de solution aqueuse,
(b) la neutralisation de ladite couche de solution organique avec une solution aqueuse alcaline pour former une couche de solution organique et une couche de solution aqueuse, et la séparation de ladite couche de solution organique de ladite couche de solution aqueuse, et à la suite
(c) l'évaporation de ladite couche de solution organique à des températures n'étant pas supérieures à 100°C et sous des pressions réduites pour obtenir la solution de (méth)acrylate de 3,4-époxycyclohexènylméthyle comprenant des ingrédients à bas point d'ébullition dans une proportion allant de 3 à 50% en poids, et ensuite
(d) l'évaporation de ladite solution de (méth)acrylate de 3,4-époxycyclohexènylméthyle à des températures n'étant pas supérieures à 100°C et à une pression inférieure à la moitié des pressions réduites de l'étape (c) pour obtenir le (méth)acrylate de 3,4-époxycyclohexènylméthyle purifié comprenant les ingrédients à bas point d'ébullition dans une proportion n'étant pas supérieure à 1% en poids.

8. Procédé selon la revendication 7, dans lequel ledit peracide organique est l'acide peracétique et ledit acide organique est l'acide acétique.

9. (méth)acrylate de 3,4-époxycyclohexylméthyle présentant une valeur au test à l'heptane inférieure à 0,1%.
